(19) Europäisches Patentamt
European Patent Office
Office européen des brevets

(11) **EP 2 021 450 B1**

(12) **FASCICULE DE BREVET EUROPEEN**

(45) Date de publication et mention de la délivrance du brevet:
**23.12.2009 Bulletin 2009/52**

(21) Numéro de dépôt: **07731895.4**

(22) Date de dépôt: **16.04.2007**

(51) Int Cl.:
*C11D 3/22* (2006.01)   *A61K 8/60* (2006.01)
*A61Q 19/10* (2006.01)   *C11D 1/66* (2006.01)
*C11D 1/29* (2006.01)

(86) Numéro de dépôt international:
**PCT/FR2007/051119**

(87) Numéro de publication internationale:
**WO 2007/125239 (08.11.2007 Gazette 2007/45)**

(54) **NOUVEAU PROCÉDÉ D'AMÉLIORATION DES PROPRIÉTÉS MOUSSANTES DE FORMULATIONS NETTOYANTES ET/OU MOUSSANTES À USAGE TOPIQUE**

NEUES VERFAHREN ZUR VERBESSERUNG DER SCHÄUMUNGSEIGENSCHAFTEN VON REINIGUNGS- UND/ODER SCHAUMMITTELN FÜR TOPISCHE ANWENDUNG

NOVEL METHOD FOR IMPROVING THE FOAMING PROPERTIES OF CLEANSING AND/OR FOAMING FORMULATIONS FOR TOPICAL USE

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **28.04.2006 FR 0651519**

(43) Date de publication de la demande:
**11.02.2009 Bulletin 2009/07**

(73) Titulaire: **Societe D'Exploitation De Produits Pour Les**
**Industries Chimiques Seppic**
**75007 Paris (FR)**

(72) Inventeurs:
• **ROSO, Alicia**
  **81710 Saix (FR)**
• **STOLTZ, Corinne**
  **94320 Thiais (FR)**

(74) Mandataire: **Conan, Philippe Claude**
**L'Air Liquide**
**Direction de la Propriété Industrielle**
**75, Quai d'Orsay**
**75321 Paris Cedex 07 (FR)**

(56) Documents cités:
**DE-A1- 4 439 091     FR-A- 1 576 613**
**US-A- 5 507 970**

**EP 2 021 450 B1**

**Description**

La présente invention a pour objet un nouveau procédé pour améliorer les propriétés moussantes de formulations nettoyantes et/ou moussantes, de nouvelles compositions et leurs procédés de préparations ainsi que la préparation des formulations à usage topique les contenant.

L'invention trouve application préférentiellement dans le domaine cosmétique et dermocosmétique, dans le domaine dermopharmaceutique et pharmaceutique, mais également dans le domaine de l'industrie textile par exemple pour le traitement de fibres textiles synthétiques ou naturelles tissées ou tricotées ou encore dans le domaine de l'industrie papetière par exemple pour la fabrication de papier à usage sanitaire ou domestique.

La mise au point de formules nettoyantes pour le visage, pour le corps et pour les cheveux, et de façon générale des produits d'hygiène corporelle et capillaire, présentées sous forme de shampoings, de lotions, de gels, de savons liquides requièrent la formation de mousse lors de l'application sur la partie du corps à nettoyer. Cette préoccupation est particulièrement importante car dans l'esprit du consommateur la création de mousse constitue une des preuves de l'efficacité du nettoyage par ces formulations. Dans le développement de formules nettoyantes pour l'hygiène corporelle et capillaire, le volume de mousse généré par la formulation, ainsi que sa stabilité et les propriétés sensorielles de ladite mousse, constituent un critère important pour le succés commercial des produits proposés aux consommateurs. Ainsi, la recherche de formulations générant une mousse de bonne qualité est également étendue à l'ensemble des produits lavant pour le corps, les gels douche et les bains moussant.

Plusieurs catégories de tensioactifs sont utilisées pour la préparation de formulations à visée nettoyante : des tensioactifs cationiques, anioniques, amphotères ou non ioniques.

Les tensioactifs anioniques, comme les tensioactifs anioniques sulfatés ou les tensioactifs de la famille des alkylcarboxylates, constituent une classe de tensioactifs fréquemment utilisée en raison de leurs bonnes propriétés moussantes. Ces tensioactifs sont réputés pour leur bon pouvoir netttoyant, et ils produisent également une mousse aérée dont le toucher n'est pas jugé désagréable par le consommateur. Cependant, ces tensioactifs présentent l'inconvénient d'être sensibles au degré de dureté de l'eau et à la présence de salissures grasses, ce qui induit par conséquence, une diminution du volume de mousse initialement généré par ces formulations mais surtout une diminution de la stabilité dans le temps de ce volume de mousse.

Pour diminuer l'ampleur de ces phénomènes, et sans toutefois les éliminer complètement, on préfère plutôt utiliser les alkyléthersulfates que les alkylsulfates. Une autre solution, elle aussi partiellement satisfaisante, consiste à utiliser des tensioactifs non ioniques, tels que les alkylpolyglycosides, comme le décylpolyglucoside ou le capryl/ caprylyl polyglucoside. Du fait de leur structure non ionique, ces tensioactifs sont compatibles avec tout autre type de tensioactifs, d'additifs et d'actifs à propriétés cosmétiques, quel que soit leur caractère anionique, cationique ou non ionique. Ils présentent notamment une excellente compatibilité avec les dérivés d'ammonium quaternaires utilisés soit pour leurs propriétés bactéricides, soit pour leur effet conditionneur des cheveux. De plus, les alkylpolyglycosides sont connus pour leur excellente capacité à former une mousse abondante, stable, indépendamment du pH de la formulation, et non sensible au degré de dureté de l'eau et à la présence de salissures grasses. Cependant, les alkylpolyglycosides produisent une mousse possédant des caractéristiques sensorielles médiocres, se traduisant par un toucher de mousse rêche, ce qui induit pendant la phase de rinçage un effet crissant particulièrement déplaisant pour le consommateur, aussi bien sur la peau que sur les cheveux.

Pour tenter de s'affranchir de ces inconvénients, il est nécessaire soit d'utiliser une faible dose d'alkylpolyglycosides dans les formulations nettoyantes, soit d'associer aux alkylpolyglycosides des agents modificateurs de toucher, comme par exemple des phases grasses hydrosolubles telles que des phases grasses siliconées ou des esters. De telles associations, si elles permettent d'améliorer les propriétés sensorielles de la mousse, ont cependant pour conséquence de diminuer de façon très significative le volume de mousse formée lors de l'utilisation par le consommateur et dans certains cas, d'altérer la stabilité de la mousse dans le temps.

Les inventeurs ont donc cherché à développer une nouvelle solution pour améliorer les propriétés moussantes des formulations cosmétiques, dermocosmétiques, dermopharmaceutiques ou pharmaceutiques.

C'est pourquoi selon un premier aspect, l'invention a pour objet un procédé pour améliorer les propriétés moussantes d'une formulation nettoyante et/ou détergente à usage topique, **caractérisé en ce que** l'on incorpore dans ladite composition, une quantité efficace d'un composé de formule (I):

$$R_1\text{-}O\text{-}(G)_x\text{-}H \qquad (I)$$

dans laquelle :

x représente un nombre décimal compris entre 1 et 5,
G représente le reste d'un sucre réducteur et
$R_1$ représente un radical monovalent de formule (A) :

$$-CH_2-(CHOH)_n-CH_2-OH \qquad (A)$$

dans laquelle n est un nombre entier égal à 2, 3 ou 4, ou bien

$R_1$ représente un radical monovalent de formule (B) :

$$-(CH_2-CHOH-CH_2-O)_m-H \qquad (B)$$

dans laquelle m est un nombre entier égal à 1, 2 ou 3,

ou d'un mélange de composés de formules (I).

Dans la définition de la formule (I) telle que définie précédemment, x est un nombre décimal qui représente le degré moyen de polymérisation du reste G. Lorsque x est un nombre entier, (G)x est le reste polymérique de rang x du reste G. Lorsque x est un nombre décimal, la formule (I) représente un mélange de composés :

$a_1$ $R_1$-O-G-H + $a_2$ $R_1$-O-(G)$_2$-H + $a_3$ $R_1$-O-(G)$_3$-H + ... + $a_q$ $R_1$-O-(G)$_q$-H avec q représentant un nombre entier compris entre 1 et 10 et dans les proportions molaires $a_1$, $a_2$, $a_3$,... $a_q$ telles que :

$$\sum_{q=10}^{q=1} a_q = 1 \; ; \; a_1 > 0$$

Selon un autre aspect particulier de la présente invention, dans la définition des composés de formules (I) x est compris entre 1,05 et 5, et plus particulièrement entre 1,05 et 2.

Par quantité efficace, on désigne, dans la définition du procédé tel que défini ci-dessus, une quantité telle, que la formulation finale, obtenue par ledit procédé :

- génère un volume de mousse supérieur ou égal à 300 cm$^3$, 30 secondes après sa formation selon les conditions opératoires du test « Ross Miles » issu de la norme ISO 696 et AFNOR NFT 73-404 dont le protocole est décrit dans le paragraphe B.1.2. de la partie expérimentale du présent exposé,
- montre une stabilité de mousse supérieure à 90%, 5 minutes après sa formation selon les conditions opératoires du test « Ross Miles ».

Selon un mode particulier du procédé tel que défini ci-dessus, par quantité efficace de composé de formule (I), on désigne une proportion massique de 0, 1 % à 20% de la formulation finale, tout particulièrement de 0,5% à 10%, et encore plus particulièrement de 1 % à 5%.

L'expression "à usage topique" utilisée dans la définition du procédé tel que défini ci-dessus, signifie que ladite composition est mise en oeuvre par application sur la peau, les cheveux, le cuir chevelu ou les muqueuses, qu'il s'agisse d'une application directe dans le cas d'une composition cosmétique, dermocosmétique, dermopharmaceutique ou pharmaceutique ou d'une application indirecte par exemple dans le cas d'un produit d'hygiène corporelle sous forme de lingette en textile ou en papier ou de produits sanitaires destinés à être en contact. avec la peau ou les muqueuses.

Par sucre réducteur, on désigne, dans la définition de la formule (I) du composé mis en en oeuvre dans le procédé tel que défini ci-dessus, les dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990.

La structure oligomérique (G)$_X$, peut se présenter sous toute forme d'isomérie, qu'il s'agisse d'isomérie optique, d'isomérie géométrique ou d'isomérie de position ; elle peut aussi représenter un mélange d'isomères.

Dans la formule (I) telle que définie ci-dessus, le groupe $R_1$-O- est lié à G par le carbone anomérique du reste saccharide, de manière à former une fonction acétal.

Selon un autre aspect particulier du procédé tel que défini ci-dessus, dans la formule (I), G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose et plus particulièrement un sucre réducteur choisi parmi le glucose, le xylose ou l'arabinose.

Selon un aspect particulier de la présente invention, celle-ci a pour objet un procédé tel que défini précédemment, dans lequel la formulation nettoyante et/ou détergente à usage topique comporte au moins un tensioactif moussant et/ou détergent.

Par tensioactif moussant et/ou détergent, on désigne les tensioactifs anioniques, cationiques, amphotères ou non ioniques topiquement acceptables habituellement utilisés dans ce domaine d'activité.

Parmi les tensioactifs anioniques que l'on peut associer à ces composés et à ces formulations, on citera particulièrement les sels de métaux alcalins, les sels de métaux alcalino-terreux, les sels d'ammonium, les sels d'amines, les sels d'aminoalcools des composés suivants : les alkyléthers sulfates, les alkylsulfates, les alkylamidoéthersulfates, les alkylarylpolyéthersulfates, les monoglycérides sulfates, les alpha-oléfinesulfonates, les paraffines sulfonates, les alkylphosphates, les alkylétherphosphates, les alkylsulfonates, les alkylamidesulfonates, les alkylarylsulfonates, les alkylcarboxylates, les alkylsulfosuccinates, les alkyléthersulfosuccinates, les alkylamidesulfosuccinates, les alkylsulfoacétates, les alkylsarcosinates, les acyliséthionates, les N-acyltaurates, les acyllactylates. Parmi les tensioactifs anioniques, on citera également les lipoaminoacides, les lipoprotéines, les lipopeptides, les dérivés des lipoprotéines, les dérivés de protéines, les sels d'acides gras, les sels d'acides d'huile de coprah éventuellement hydrogénée.

Parmi les tensioactifs amphotères que l'on peut associer à ces composés et à ces formulations, on citera particulièrement les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques que l'on peut associer à ces composés et à ces formulations, on citera particulièrement les dérivés d'ammoniums quaternaires.

Parmi les tensioactifs non ioniques que l'on peut associer à ces composés et à ces formulations, on citera particulièrement les alkylpolyglycosides, les dérivés d'huile de ricin, les polysorbates, les amides de coprah, les N-alkylamines, les oxydes d'amines.

Parmi les tensioactifs moussants et/ou détergents cités ci-dessus, qui sont des tensioactifs anioniques, il y a plus particulièrement les composés de formule (II) :

$$[R_2\text{-O-}(CH_2\text{-}CH_2\text{-O})_p SO_3]_r X \qquad (II)$$

dans laquelle:

$R_2$ représente un radical hydrocarboné aliphatique, saturé ou insaturé, linéaire ou ramifié, comportant de 6 à 22 atomes de carbone,

p représente un nombre décimal compris entre 1 et 10, de préférence entre 2 et 4,

r est un nombre entier égal à 1 ou à 2 et

X représente le cation d'un métal alcalin ou d'un métal alcalino-terreux, l'ion ammonium, l'ion hydroxyéthyl ammonium, l'ion tris(hydroxyéthyl) ammonium.

Dans la formule (II) telle que définie ci-dessus, X représente par exemple le sodium, le magnésium ou le l'ion ammonium.

Parmi les tensioactifs moussants et/ou détergents cités ci-dessus, qui sont des tensioactifs non-ioniques, il y a plus particulièrement les composés de formule (III) :

$$R_3\text{-O-}(S)_y\text{-H} \qquad (III)$$

dans laquelle :

y représente un nombre décimal compris entre 1 et 5,

S représente le reste d'un sucre réducteur et

$R_3$ représente un radical alkyle linéaire ou ramifié, saturé ou insaturé, ayant de 8 à 16 atomes de carbone, de préférence de 8 à 14 atomes de carbone.

Dans la définition de la formule (III) telle que définie précédemment, y est un nombre décimal qui représente le degré moyen de polymérisation du reste S. Lorsque y est un nombre entier, $(S)_y$ est le reste polymérique de rang y du reste S ; lorsque y est un nombre décimal, la formule (III) représente un mélange de composés :

$a_1 R_3\text{-O-S-H} + a_2 R_3\text{-O-}(S)_2\text{-H} + a_3 R_3\text{-O-}(S)_3\text{-H} + ... + a_q R_3\text{-O-}(S)_q\text{-H}$ avec q représentant un nombre entier compris entre 1 et 10 et dans les proportions molaires $a_1$, $a_2$, $a_3$,... $a_q$ telles que :

$$\sum_{q=10}^{q=1} a_q = 1 \; ; \; a_1 > 0$$

Selon un autre aspect particulier de la présente invention, dans la définition des composés de formules (III) y est compris entre 1,05 et 5, et plus particulièrement entre 1,05 et 2.

Dans la formule (III) telle que définie ci-dessus, $R_3$ représente par exemple le radical n-octyle, le radical n-décyle, le radical n-dodécyle, le radical n-dodécyle ou le radical n-tétradécyle.

Par sucre réducteur, on désigne, dans la définition de la formule (III) du composé mis en en oeuvre dans le procédé tel que défini ci-dessus, les dérivés saccharidiques qui ne présentent pas dans leurs structures de liaison glycosidique établie entre un carbone anomérique et l'oxygène d'un groupement acétal tels qu'ils sont définis dans l'ouvrage de référence :

« Biochemistry », Daniel Voet/Judith G. Voet, p. 250, John Wyley & Sons, 1990. La structure oligomérique $(S)_y$ peut se présenter sous toute forme d'isomérie, qu'il s'agisse d'isomérie optique, d'isomérie géométrique ou d'isomérie de position ; elle peut aussi représenter un mélange d'isomères.

Dans la formule (III) telle que définie ci-dessus, le groupe $R_3$-O- est lié à S par le carbone anomérique du reste saccharide, de manière à former une fonction acétal.

Selon un autre aspect particulier du procédé tel que défini précédemment, le rapport massique composés de formule (I) sur agents tensioactifs moussants et/ou détergents présents dans ladite composition à usage topique, est compris entre 1/30 et 10/1 plus particulièrement entre 1/30 et 1/1.

Selon un deuxième aspect l'invention a pour objet une composition (C) comprenant pour 100% de sa masse :

- de 97% à 40% massique d'un composé de formule (III) :

$$R_3\text{-O-}(S)_y\text{-H} \qquad \text{(III)}$$

dans laquelle :

y représente un nombre décimal compris entre 1 et 5,
S représente le reste d'un sucre réducteur et
$R_3$ représente un radical alkyle linéaire ou ramifié, saturé ou insaturé, ayant de 8 à 16 atomes de carbone, de préférence de 8 à 14 atomes de carbone,

ou d'un mélange de composés de formule (III) ;
de 1% à 25% massique d'un composé de formule (I) :

$$R_1\text{-O-}(G)_x\text{-H} \qquad \text{(I)}$$

dans laquelle :

x représente un nombre décimal compris entre 1 et 5,
G représente le reste d'un sucre réducteur et
$R_1$ représente un radical monovalent de formule (A) :

$$\text{-CH}_2\text{-(CHOH)}_n\text{-CH}_2\text{-OH} \qquad \text{(A)}$$

dans laquelle n est un nombre entier égal à 2, 3 ou 4, ou bien
$R_1$ représente un radical monovalent de formule (B) :

$$\text{-(CH}_2\text{-CHOH-CH}_2\text{-O)}_m\text{-H} \qquad \text{(B)}$$

dans laquelle m est un nombre entier égal à 1, 2 ou 3,

ou d'un mélange de composés de formules (I) ; et

jusqu'à 50 % massique d'un solvant topiquement acceptable.

Par solvant topiquement acceptable, on désigne dans le cadre de la présente invention les solvants connus de l'homme du métier pouvant être appliqués sur la peau humaine et/ou animale, sur le cuir chevelu et sur les muqueuses.

Selon un premier aspect particulier de la composition (C), dans la formule (I) G représente le reste de sucre réducteur choisi parmi le glucose, le xylose ou l'arabinose.

Selon un deuxième aspect particulier de la composition (C), dans la formule (I) $R_1$ représente un radical monovalent de formule (A) pour lequel n est égal à 2 ou à 3, ou un radical monovalent de formule (B) pour lequel m est égal à 1 ou à 2.

Selon un troisième aspect particulier de la composition (C), dans la formule (III) S représente le reste de sucre réducteur choisi parmi le glucose, le xylose ou l'arabinose.

Selon un quatrième aspect particulier de la composition (C), dans la formule (III) $R_3$ représente un radical choisi parmi les radicaux n-octyle, n-décyle, n-dodécyle, n-tétradécyle, n-hexadécyle.

Le solvant topiquement acceptable est plus particulièrement choisi parmi un ou plusieurs éléments d'un groupe constitué par l'eau, des glycols, des polyols, des alcools, des polyols alcoxylés, des éthers de glycols.

Dans le cadre de la présente invention, les solvants topiquement acceptables sont choisis tout particulèrement parmi un ou plusieurs éléments d'un groupe constitué par l'eau, l'éthanol, l'isopropanol, le butylène glycol, l'hexylène glycol, le caprylyl glycol ou 1,2 octanediol, le pentylene glycol ou 1,2 pentanediol, l'éthylhexylglycérine ou octoxyglycérine, le glycérol, le diglycérol, le triglycérol, l'érythritol, le xylitol, le sorbitol, le butyldiglycol, les polyéthylènes glycols dont le poids moléculaire est compris entre 200 g.mol$^{-1}$ et 8000 g.mol$^{-1}$, le monopropylène glycol, le dipropylène glycol, le butyldiglycol, le méthyl-2,-propanediol-1,3.

Avantageusement, le solvant topiquement acceptable précité est choisi parmi l'eau, et parmi un ou plusieurs éléments du groupe des polyols constitué par le xylitol, l'érythritol, le sorbitol, le glycérol et le diglycérol.

Les composés de formule (III) ou les mélanges de composés de formule (III), les composés de formule (I) ou les mélanges de composés de formule (I) et le solvant topiquement acceptable peuvent être incorporés dans la formulation cosmétique, à usage topique de façon séparée ou bien sous la forme d'une composition (C) objet de l'invention. D'autre part, selon une ou plusieurs voies de préparation des composés de formule (I) ou des mélanges de composés de formule (I), qui consiste à faire réagir un sucre réducteur G avec un polyol de formule (A1) :

$$HO-CH_2-(CHOH)_n-CH_2-OH \qquad (A1)$$

dans laquelle n est un nombre entier égal à 2, 3 ou 4 et/ou de formule (B1),

$$HO-(CH_2-CHOH-CH_2-O)_m-H \qquad (B1)$$

dans laquelle m est un nombre entier égal à 1, 2 ou 3,

la quantité de polyol n'ayant pas réagi, lorsque celui-ci a été sélectionné parmi un ou plusieurs éléments du groupe défini précédemment, peut constituer toute ou partie du solvant topiquement acceptable. Dans ce cas, les composés de formule (I) ou les mélanges de composés de formule (I) et le solvant topiquement acceptable sont incorporés dans la formulation cosmétique, à usage topique de façon concomitante et les composés de formule (III) peuvent être incorporés dans une étape ultérieure.

Selon les procédés utilisés pour préparer la composition (C) selon l'invention, (C) peut comprendre de façon résiduelle des composés secondaires résultant de la préparation des composés de formule (I), comme les polyols de formule (A1) ou les polyols de formule (B1) sous leurs formes déshydratées. La composition (C) ne peut pas comprendre plus de 10 % de ces composés secondaires.

La composition (C) objet de l'invention peut être obtenue par diverses voies :

Une première voie de synthèse consiste en une première étape (a), à introduire un composé de formule (I) ou un mélange de composés de formule (I) et un composé de formule (III) ou un mélange de composés de formule (III) dans un réacteur selon un rapport massique maîtrisé, et à soumettre ce mélange à une agitation mécanique efficace, dans des conditions de température permettant d'assurer l'homogénéité du mélange, préférentiellement entre 20°C et 90°C. Si nécessaire, une seconde étape (b) consiste à introduire un solvant topiquement acceptable tel que défini précédemment au mélange obtenu lors de l'étape (a), et à poursuivre l'agitation jusqu'à l'obtention d'une composition homogène.

Une deuxième voie de synthèse de la composition (C) selon l'invention consiste à synthétiser lors d'une première étape (a1), le composé de formule (I) ou le mélange de composés de formule (I) en introduisant un sucre réducteur et un polyol de formule (A1) ou (B1) tels que par exemple l'érythritol, le xylitol, le glycérol, le diglycérol, le triglycérol ou le sorbitol, dans un réacteur, selon un rapport stoechiométrique maîtrisé, et à soumettre ce mélange à une réaction d'acétalisation dans des conditions de température et de vide partiel prédéterminées en présence d'un système catalytique acide. Les composants de ce système catalytique acide seront généralement choisis parmi les acides sulfurique, chlorhydrique, phosphorique, nitrique, hypophosphoreux, méthane-sulfonique, para- toluène sulfonique, trifluoro-méthane sulfonique et les résines échangeuses d'ions acides. Habituellement, la réaction d'acétalisation sera réalisée à une température de 70 à 130°C, sous un vide de 300 à 20 $10^2$ Pa (300 à 20 mbars). Lors d'une deuxième étape (b1), un composé de formule (III) ou un mélange de composés de formule (III) est mélangé avec le produit de la réaction obtenu lors de l'étape (a1), par l'intermédiaire d'un système d'agitation permettant d'atteindre une composition homogène. Si nécessaire, une troisième étape (c1) consiste à introduire un solvant topiquement acceptable tel que défini précédemment au mélange obtenu lors de l'étape (b1), et à poursuivre l'agitation jusqu'à l'obtention d'une composition homogène.

Une troisième voie de synthèse consiste à soumettre le polyol de formule (A1) ou (B1) à une déshydratation, en présence d'un système catalytique acide, à une température comprise entre 70°C et 130°C, sous vide partiel, avec élimination concomitante de l'eau formée lors du réarrangement intra-moléculaire subi par le polyol lors d'une première étape (a2); puis à acétaliser le polyol déshydraté ainsi obtenu par dispersion d'un sucre réducteur dans le milieu réactionnel et par maintien de celui-ci à une température comprise entre 80°C et 130°C, sous vide partiel, lors d'une deuxième étape (b2). Le système catalytique acide utilisé dans cette troisième voie de synthèse peut être identique à celui évoqué pour la deuxième voie. Si nécessaire, une troisième étape (c2) consiste à introduire un solvant topiquement acceptable tel que défini précédemment au mélange obtenu lors de l'étape (b2), et à poursuivre l'agitation jusqu'à l'obtention d'une composition homogène.

Une quatrième voie de synthèse par trans-acétalisation consiste à préparer du butylglucoside par réaction entre le butanol et le glucose en présence d'un système catalytique acide, à une température comprise entre 90°C et 105°C, sous vide partiel, avec élimination concomitante de l'eau formée lors de la réaction lors d'une première étape (a3), le système catalytique acide utilisé pouvant être identique à celui évoqué pour les voies de synthèse précédentes ; ajouter un polyol de formule (A1) ou (B1) au milieu réactionnel ainsi obtenu, avec évacuation par distillation sous vide du butanol résiduel, du butanol formé au cours de la réaction de trans-acétalisation, et de l'eau éventuellement générée lors du réarrangement intra-moléculaire dudit polyol lors d'une deuxième étape (b3) ; et si nécessaire, une troisième étape (c3) consiste à introduire un solvant topiquement acceptable tel que défini précédemment au mélange obtenu lors de l'étape (b3), et à poursuivre l'agitation jusqu'à l'obtention d'une composition homogène.

Selon un troisième aspect, l'invention telle que définie précédemment a pour objet l'utilisation d'un composé de formule (I), telle que définie précédemment, ou d'un mélange de composés de formules (I) ou l'utilisation d'une composition (C) telle que définie précédemment, pour améliorer les propriétés moussantes d'une formulation cosmétique, dermocosmétique, dermopharmaceutique ou pharmaceutique à usage topique.

Selon un quatrième aspect, l'invention a pour objet la préparation d'une formulation nettoyante et/ou moussante à usage topique par la mise en oeuvre d'une quantité efficace d'une composition (C) telle que définie précédemment. Par quantité efficace, on désigne une proportion massique de la composition (C) précédemment définie dans la formulation nettoyante et/ou détergente comprise entre 0.5% et 50 %, plus particulièrement entre 1% et 30% et tout particulièrement entre 2% et 10% de la masse totale de ladite formulation.

Les composés de formule (I), les mélanges de composés de formule (I) ainsi que la composition (C) telle que définies précédemment, peuvent être incorporés dans tout type de formulation cosmétique, dermocosmétique, dermopharmaceutique ou pharmaceutique destinée à un usage topique, ou bien encore dans tout type de support destiné à être mis en contact avec la peau (papier, lingette, textile, dispositif transdermique, etc.). Les formulations cosmétiques, à usage topique dans lesquelles est incorporée une quantité efficace de composés de formule (I) ou de mélanges de composés de formule (I), et qui contiennent éventuellement un ou plusieurs tensioactifs moussants et/ou détergents, ou bien la composition (C) précédemment définie, peuvent être appliquées indifféremment sur la peau, sur les cheveux, sur le cuir chevelu et sur les muqueuses, et se présentent notamment sous la forme d'une solution aqueuse ou huileuse, d'une émulsion ou d'une micro-émulsion du type eau dans huile (E/H) ou huile-dans-eau (H/E), d'une émulsion multiple de type eau-dans-huile-dans-eau (E/H/E) ou huile-dans-eau-dans-huile (H/E/H), d'un gel, d'un savon ou d'un syndet, d'un baume, d'une hydro-dispersion, d'une crème, d'une mousse, d'un aérosol ou encore sous forme anhydre comme une poudre. Ces formulations peuvent être utilisées comme laits nettoyants ou démaquillants, comme lotions nettoyantes ou démaquillantes, comme gels moussants pour le visage ou pour le corps, comme shampooing ou après-shampooing, comme bain moussant.

De façon générale ces formulations comportent, en plus des tensioactifs moussants et/ou détergents qui peuvent y être éventuellement présents, des excipients et ou des principes actifs habituellement mis en oeuvre dans le domaine des formulations à usage topique, en particulier cosmétiques, dermocosmétiques, pharmaceutiques ou dermopharmaceutiques, les épaississants, les gélifiants, les stabilisants, les composés filmogènes, les solvants et co-solvants, les agents hydrotropes, les agents plastifiants, les matières grasses, les huiles, les agents émulsionnants et co-émulsionnants, les agents opacifiants, les agents nacrants, les agents surgraissants, les séquestrants, les agents chélatants, les antioxydants, les parfums, les conservateurs, les agents conditionneurs, les agents blanchissants destinés à la décoloration des poils et de la peau, les principes actifs destinés à apporter une action traitante vis à vis de la peau ou des cheveux, les filtres solaires, les charges minérales ou les pigments, les particules procurant un effet visuel ou destinées à l'encapsulation d'actifs, les particules exfoliantes, les agents de texture, les azurants optiques, les repellents pour les insectes.

Comme exemples de polymères épaississants et/ou gélifiants éventuellement présents dans la formulation pour laquelle on met en oeuvre le procédé objet de la présente invention, on peut citer :

- les homopolymères ou copolymères de l'acide acrylique ou de dérivés de l'acide acrylique, les homopolymères ou copolymères de l'acrylamide, les homopolymères ou copolymères de dérivés de l'acrylamide, les homopolymères ou copolymères de l'acide acrylamidométhyl propanesulfonique, de monomère vinylique et/ou de chlorure de triméthylaminoéthylacrylate commercialisés sous les noms CARBOPOL™, ULTREZ™ 10, ACULYN™ , PEMULEN™ TR1, PEMULENTM TR2, SIMULGEL™ EG, SIMULGEL™ EPG LUVIGEL™ EM, SALCARE™ SC91, SALCARE™ SC92, SALCARE™ SC95, SALCARE™ SC96, FLOCARE™ ET 100, FLOCARE™ ET58, HISPAGEL™, SEPIGEL™ 305, SEPIGEL™ 501, SEPIGEL™ 502, SIMULGEL™ NS, SIMULGEL™ 800, SIMULGEL™ A, SEPIPLUS™ 250, SEPIPLUS™ 265, SEPIPLUS™ 400, SEPINOV™ EMT 10, NOVEMER™ EC1, ARISTOFLEX™ AVC, ARISTOFLEX™ HBM, RAPITHIX™ A60, RAPITHIX™ A100, COSMEDIA™ SP et STABILEZE™ 06 ;
- les hydrocolloïdes d'origine végétale ou biosynthétique, par exemple la gomme de xanthane, la gomme de karaya, les carraghénates, les alginates, les galactomannanes ;
- les silicates ; la cellulose et ses dérivés ; l'amidon et ses dérivés hydrophiles ; les polyuréthanes.

Comme exemples de tensioactifs épaississants et/ou gélifiants éventuellement présents dans la formulation pour laquelle on met en oeuvre le procédé objet de la présente invention, on peut citer :

- les esters gras d'alkylpolyglycosides éventuellement alkoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120
- Les esters gras alkoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141.
- les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.

Comme exemples d'émulsionnants éventuellement présents dans la formulation pour laquelle on met en oeuvre le procédé objet de la présente invention, on peut citer :

- les acides gras, les acides gras éthoxylés, les esters d'acide gras et de sorbitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435, 2 804 432, 2 830 774, 2 830 445, les associations de tensioactifs émulsionnants choisis parmi les alkylpolyglycosides, les associations d'alkylpolyglycosides et d'alcools gras, les esters de polyglycérols ou de polyglycols ou de polyols tels que les polyhydroxystéarates de polyglycols ou de polyglycérols mis en oeuvre dans les demandes de brevets français 2 852 257, 2 858 554, 2 820 316 et 2 852 258.

Comme exemples d'agents opacifiants et/ou nacrants éventuellement présents dans la formulation pour laquelle on met en oeuvre le procédé objet de la présente invention, on peut citer les palmitates ou les stéarates ou les hydroxystéarates de sodium ou de magnésium, les monostéarates ou distéarates d'éthylène ou de polyéthylène glycol, les alcools gras, les homopolymères et copolymères de styrène tels que le styrène acrylate copolymère commercialisé sous l'appellation MONTOPOL™ OP1 par la société SEPPIC.

Comme exemples d'huiles éventuellement présents dans la formulation pour laquelle on met en oeuvre le procédé objet de la présente invention,
on peut citer :

- les huiles minérales telles que l'huile de paraffine, l'huile de vaseline, les isoparaffines ou les huiles blanches minérales ;
- les huiles d'origine animale, telles que le squalène ou le squalane,
- les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;

- les huiles végétales éthoxylées ;
- les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les polyalphaoléfines, les polyoléfines comme le polyisobutène, les isoalcanes de synthèse comme l'isohexadécane, l'isododécane, les huiles perfluorées et
- les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Comme autre matière grasse éventuellement présente dans la formulation pour laquelle on met en oeuvre le procédé objet de la présente invention, on peut citer les alcools gras ou les acides gras ; les cires telles que la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène, les cires de silicone ; les cires végétales ; les alcools gras et les acides gras solides à température ambiante ; les glycérides solides à température ambiante.

Comme exemples de principes actifs éventuellement présents dans la formulation pour laquelle on met en oeuvre le procédé objet de la présente invention, on peut citer les composés ayant une action éclaircissante ou dépigmentante, une action hydratante, une action tenseur, une action apaisante ou relaxante, une action anti inflammatoire, une action amincissante, une action lipolytique, une action drainante, une action détoxifiante, une action une action énergisante, décontractante, une action stimulante, une action émoliente, une action neuromodulatrice, une action protectrice, une action purifiante, séborégulatrice, antichute, une action anti-age, une action raffermissante, restructurante, antiradicalaire, ou antioxydante ; de tels principes actifs sont par exemple le SEPIWHITE™MSH, l'arbutine, l'acide kojique, l'hydroquinone, l'acide ellagique, la vitamine C et ses dérivés, le Stay C, le magnésium ascorbyl phosphate et ses dérivés, l'acorbyl glucoside, l'acide phytique, les acides de fruits, le rucinol ou resorcinol, l'acide azélaïque, l'acide lipoïque, le VEGEWHITE™, la gGATULINE™, le SYNERLIGHT™, le BIOWHITE™, le PHYTOLIGHT™, le DERMALIGHT™, la CLARISKIN™, le MELASLOW™, le DERMAWHITE™, l'ETHIOLINE, le MELAREST™, le GIGAWHITE™, l'ALBATINE™, le LUMISKIN™, les extraits de polyphénols, les extraits de raisin, les extraits de pin, les extraits de vin, les extraits d'olives, les extraits de mare, les protéines N-acylées, les peptides N-acylés comme par exemple le MATRIXIL™, les acides aminés N - acylés, les hydrolysâts partiels de protéines N-acylés, les acides aminés, les peptides, les hydrolysâts totaux de protéines, les polyols (par exemple, la glycérine ou le butylène glycol), les dérivés de lait, ou les différents composants entrant dans la composition du NMF (natural moisturizing factor) par exemples l'urée, l'acide pyrrolidone carboxylique ou les dérivés de cet acide, les acides aminés, les sels minéraux, les glucosamines, l'acide glycyrrhétinique, l'alpha-bisabolol, les sucres ou les dérivés des sucres, les polysaccharides ou leurs dérivés, les hydroxyacides par exemple l'acide lactique, les vitamines, les dérivés de vitamines, par exemple le Rétinol, la vitamine E et ses dérivés, les oligo-éléments, les extraits de roches ou pierres, les enzymes, les co-enzymes, comme, le Coenzyme Q10, les hormones ou "hormone like" comme par exemple le PHYTO AGE™, les extraits de soja, par exemple la Raffermine™, les extraits de blé par exemple la TENSINE™ ou la GLIADINE™, les extraits végétaux, tels que les extraits végétaux riches en tanins, les extraits végétaux riches en isoflavones ou les extraits végétaux riches en terpènes, les extraits d'algues d'eau douce ou marines, les extraits marins en général comme les coraux, les cires essentielles, les extraits bactériens, les minéraux comme les GIVOBIO™, les dérivés de calcium, de magnésium, de cuivre, de cobalt, de zinc, de lithium, ou de manganèse, les sels d'argent ou d'or, les lipides en général, les lipides tels que les céramides ou les phospholipides, les actifs ayant une action amincissante ou lipolytique comme la caféine ou ses dérivés, le calcium et ses dérivés, le LIPASLIM™, les actifs améliorant la microcirculation cutanée, par exemple les veinotoniques, les actifs drainants, les actifs à visée décongestionnante tels que le ginko biloba, le lierre, le marron d'inde, le bambou, le ruscus, le petit houx, la centalla asiatica, le fucus le romarin, le saule, les actifs ayant une activité antimicrobienne ou une action purifiante vis à vis des peaux grasses, par exemple, le LIPACIDE™ C8G, le LIPACIDE™ UG, le SEPICONTROL™ A5, les dérivés de cuivre ou de zinc, l'OCTOPIROX™ ou le SENSIVA™ SC50, les actifs ayant une propriété énergisante ou stimulante comme le SEPITONIC™ M3 ou le Physiogényl™ le panthénol et ses dérivés comme le SEPICAP™ MP, les actifs anti-age comme le SEPILIFT™ DPHP, le LIPACIDE™ PVB, le SEPIVINOL™, le SEPIVITAL™, le MANOLIVA™, le PHYTO-AGE™, les actifs hydratants comme le SEPICALM™ S, le SEPICALM™ VG et le LIPACIDE™ DPHP, les actifs anti-photo vieillissement ", les actifs protecteurs de l'intégrité de la jonction dermo-épidermique, les actifs augmentant la synthèse des composants de la matrice extracellulaire par exemple, le collagène, les élastines, les glycosaminoglycanes, les actifs agissant favorablement sur la communication cellulaire chimique comme les cytokines ou physiques comme les intégrines, les actifs créant une sensation de « chauffe » sur la peau comme les activateurs de la microcirculation cutanée (exemple des nicotinates) ou des produits créant une sensation de « fraîcheur » sur la peau (exemple du menthol et des dérivés).

Comme filtres solaires éventuellement présents dans la formulation pour laquelle on met en oeuvre le procédé objet de la présente invention,, on peut citer tous ceux figurant dans la directive cosmétique 76/768/CEE modifiée annexe VII.

Les exemples suivants illustrent l'invention, sans toutefois la limiter.

## A)- Préparation des composés de formule (I) ou de mélanges de composés de formule (I), et des compositions selon l'invention

EXEMPLE 1 : Composition (C1) constituée de xylitylpolyglucosides, de lauryléther (2,2 OE) sulfate de sodium et de Xylitol

On introduit 703,0g de xylitol dans un réacteur en verre à double enveloppe, dans laquelle circule un fluide caloporteur, et muni d'une agitation efficace. Le xylitol est fondu à une température de 135°C, et la pâte visqueuse ainsi obtenue est refroidie à 115°C. Le glucose est alors ajouté progressivement au milieu réactionnel pour permettre sa dispersion homogène. On ajoute au mélange ainsi obtenu un système catalytique acide constitué de 1,29g d'acide sulfurique à 96%. Le milieu réactionnel est placé sous un vide partiel de 90 $10^2$ Pa (90 mbars) à 45 $10^2$ Pa (45 mbars) et maintenu à une température de 100°C-105°C pendant une durée de 4h 30 avec évacuation de l'eau formée au moyen d'un montage de distillation. Le milieu réactionnel est ensuite refroidi à 95°C-100°C et neutralisé par ajout de 5g de soude à 30%, pour amener le pH d'une solution à 1% de ce mélange à une valeur de 5,0. Les caractéristiques du mélange intermédiaire ainsi obtenu sont les suivantes :

Aspect (visuel) : cire orange à température ambiante ;
pH solution à 1 % : 5,0 ;
xylitol résiduel : 56% massique;
glucose résiduel : < 1 % massique ;
Xylityl polyglucosides : 38% massique.

15,86 g du mélange intermédiaire précédemment obtenu et 572,9g de lauryléther(2,2 OE) sulfate de sodium à 28% dans l'eau sont mélangés à 50°C dans un réacteur en verre à double enveloppe, dans laquelle circule un fluide caloporteur, et muni d'une agitation efficace.

Après obtention d'un mélange homogène, la composition (C1) comporte pour 100% massique de sa matière sèche, 91% de lauryléther(2,2 OE) sulfate de sodium, 4% de xylityl polyglucoside et 5% de xylitol.

EXEMPLE COMPARATIF 1 : Lauryléther (2,2 OE) sulfate de sodium (T1)

EXEMPLE 2 : Composition (C2) constituée de xylitylpolyglucosides, de n-décyl/n-dodécyl polyglucosides et de xylitol.

Les xylitylpolyglucosides sont préparés selon le procédé décrit dans l'exemple 1 jusqu'à l'obtention d'un mélange intermédiaire comprenant pour 100% de sa masse, 37,2% massique de xylitylpolyglucosides, 55,8% massique de xylitol et une proportion massique de glucose résiduelle inférieure à 1%. 20g du mélange intermédiaire précédemment obtenu et 113,3g d'un mélange de n-décylpolyglucosides et de n-dodécylpolyglucosides (C10/C12 = 85/15) en solution à 55% sont mélangés à 50°C dans un réacteur en verre à double enveloppe, dans laquelle circule un fluide caloporteur, et muni d'une agitation efficace. Après obtention d'un mélange homogène, la composition (C2) comporte pour 100% massique de sa matière sèche, 77% de n-décylpolyglucosides/n-dodécylpolyglucosides, 9% de Xylitylpolyglucosides et 14% de Xylitol.

EXEMPLE COMPARATIF 2 : Composition (T2) constituée de n-décyl / n-dodécyl polyglucosides et de xylitol.

On introduit de la solution à 55% de n-décylpolyglucosides et de n-dodécylpolyglucosides (C10/C12 = 85/15) dans un bêcher à température ambiante. Le milieu est agité par un barreau aimanté couplé à un agitateur magnétique

EP 2 021 450 B1

et le xylitol est introduit progressivement dans des proportions permettant d'atteindre une composition contenant 77% massique de matière sèche de n-décylpolyglucosides/n-dodécylpolyglucosides et 23% massique de xylitol.

EXEMPLE 3 : Composition (C3) constituée par des xylitylpolyglucosides, des n-octyl/n-décyl polyglucosides et du xylitol.

Les xylitylpolyglucosides sont préparés selon le procédé décrit dans l'exemple 1 jusqu'à l'obtention d'un mélange intermédiaire comprenant pour 100% de sa masse 37,2% massique de xylitylpolyglucosides, 55,8% massique de xylitol et une proportion massique de glucose résiduelle inférieure à 1%. 20g du mélange intermédiaire précédemment obtenu et 103,8g d'un mélange de n-octylpolyglucosides et de n-décylpolyglucosides (C8/C10 = 50/50) en solution à 60% sont mélangés à 50°C dans un réacteur en verre à double enveloppe, dans laquelle circule un fluide caloporteur, et muni d'une agitation efficace. Après obtention d'un mélange homogène, la composition massique de la composition (C3) comporte pour 100% de sa matière sèche, 77% de n-octylpolyglucosides/n-décylpolyglucosides, 9% de Xylitylpolyglucosides et 14% de Xylitol.

EXEMPLE COMPARATIF 3 : Solution à 55% en matière sèche de n-décylpolyglucosides/n-dodécylpolyglucosides (T3).

EXEMPLE 4 : Composition (C4) constituée par des xylitylpolyglucosides, des n-octyl/n-décyl polyglucosides et du xylitol.

Les xylitylpolyglucosides sont préparés selon le procédé décrit dans l'exemple 1 jusqu'à l'obtention d'un mélange intermédiaire comprenant pour 100% de sa masse 37,2% massique de xylitylpolyglucosides, 55,8% massique de xylitol et une proportion massique de glucose résiduelle inférieure à 1%. 20g du mélange intermédiaire précédemment obtenu et 62,1g d'un mélange de n-octylpolyglucosides et de n-décylpolyglucosides en solution à 60% sont mélangés à 50°C dans un réacteur en verre à double enveloppe, dans laquelle circule un fluide caloporteur, et muni d'une agitation efficace. Après obtention d'un mélange homogène, la composition (C4) comporte pour 100% de sa matière sèche de 68% de n-octylpolyglucosides/n-décylpolyglucosides, de 7% de Xylityl polyglucosides et de 25% de Xylitol.

EXEMPLE COMPARATIF 4 : Solution à 55% en matière sèche de n-octylpolyglucosides/n-décylpolyglucosides (T4).

EXEMPLE 5 : Composition (C5) constituée par des diglycérylpolyxylosides, des n-octyl/n-décyl polyglucosides et du diglycérol

On introduit 650,0g de diglycérol dans un réacteur en verre à double enveloppe, dans laquelle circule un fluide caloporteur, et muni d'une agitation efficace. Le diglycérol est amené à une température d'environ 100°C. 1117,5 g de xylose sont alors ajoutés progressivement au milieu réactionnel pour permettre sa dispersion homogène. On ajoute au mélange ainsi obtenu un système catalytique acide constitué de 0,51g d'acide sulfurique à 98%. Le milieu réactionnel est placé sous un vide partiel à $30 \cdot 10^2$ Pa (30 mbars) mbars, et maintenu à une température de 100°C-105°C pendant une durée de 4h 00 avec évacuation de l'eau formée au moyen d'un montage de distillation. Le milieu réactionnel est ensuite refroidi à 95°C-100°C et neutralisé par ajout de soude à 30%, pour amener le pH d'une solution à 1% de ce mélange à une valeur d'environ 7,0. Les caractéristiques du mélange intermédiaire ainsi obtenu sont les suivantes :

Aspect (visuel) : liquide limpide;
pH solution à 1% : 6,8 ;
Diglycérol résiduel : 68,1% ;
Xylose résiduel : < 1 %.
Diglycéryl polyxylosides : 27,7%

20g du mélange intermédiaire précédemment obtenu et 133,3g d'un mélange de n-octylpolyglucosides et de n-décylpolyglucosides en solution à 60% sont mélangés à 50°C dans un réacteur en verre à double enveloppe, dans laquelle circule un fluide caloporteur, et muni d'une agitation efficace. Après obtention d'un mélange homogène, la composition (C5) comporte pour 100% de sa matière sèche de 80% de n-octylpolyglucosides/n-décylpolyglucosides, de 6% de Diglycérylpolyxylosides et de 14% de Diglycérol.

EXEMPLE 6 : Composition (C6) constituée par des diglycérylpolyglucosides, des n-dodécyl/n-tétradécyl/n-hexadécyl polyglucosides et du diglycérol.

Le procédé décrit dans l'exemple 5 est mis en oeuvre pour préparer les diglycérylpolyglucosides en substituant le xylose par le glucose et en conservant le même rapport molaire sucre réducteur/diglycérol égal à 1/5. Les caractéristiques du mélange intermédiaire ainsi obtenu sont les suivantes :

Aspect (visuel) : liquide limpide;
pH solution à 1 % : 6,8 ;
Diglycérol résiduel : 67,2% ;
Glucose résiduel : < 1%.
Diglycéryl polyglucosides : 24,7%

20g du mélange intermédiaire précédemment obtenu et 120g d'un mélange de n-décylpolyglucosides, n-dodécylpolyglucosides, de n-tétracylpolyglucosides et de n-hexadécylpolyglucosides commercialisés sous l'appellation PLANTACARE™ 1200 UP sont mélangés à 50°C dans un réacteur en verre à double enveloppe, dans laquelle circule un fluide caloporteur, et muni d'une agitation efficace. Après obtention d'un mélange homogène, la composition (C6) comporte pour 100% de sa matière sèche 75% d'un mélange de n-décyl polyglucosides, n-dodécyl polyglucosides, n-tétradécyl polyglucosides, n-hexadécyl polyglucosides, 8% de Diglycérylpolyglucosides et 17% de Diglycérol.

TABLEAU RECAPITULATIF

Le tableau 1 ci-dessous récapitule les compositions massiques en matières sèches des différentes compositions préparées et destinées à être mises en oeuvre dans des tests de mise en évidence des propriétés moussantes.

Tableau 1

| composition (en % massique) | C1 | T1 | C2 | T2 | C3 | T3 | C4 | T4 | C5 | C6 |
|---|---|---|---|---|---|---|---|---|---|---|
| Lauryléther (2,2 OE) sulfate de sodium | 91 | 100 | 0 | 0 | 0 | 0 | 0 | 0 | 0 | 0 |
| Xylityl polyglucosides | 4 | 0 | 9 | 0 | 9 | 0 | 7 | 0 | 0 | 0 |
| Xylitol | 5 | 0 | 14 | 23 | 14 | 0 | 25 | 0 | 0 | 0 |
| Diglycéryl polyxylosides | 0 | 0 | 0 | 0 | 0 | 0 | 0 | 0 | 6 | 0 |
| Diglycéryl polyglucosides | 0 | 0 | 0 | 0 | 0 | 0 | 0 | 0 | 0 | 8 |
| Diglycérol | 0 | 0 | 0 | 0 | 0 | 0 | 0 | 0 | 14 | 17 |
| n-octyl/n-décyl polyglucosides | 0 | 0 | 0 | 0 | 77 | 0 | 68 | 100 | 80 | 0 |
| n-décyl/n-dodécyl polyglucosides | 0 | 0 | 77 | 77 | 0 | 100 | 0 | 0 | 0 | 0 |
| n-décyl/n-dodécyl/n-tétradécyl/n-hexadécyl polyglucosides | 0 | 0 | 0 | 0 | 0 | 0 | 0 | 0 | 0 | 75 |

**B - Mise en évidence des propriétés moussantes des formulations comprenant des composés de formule (I) ou des mélange de composés de formule (I), et des compositions (C) selon l'invention.**

La mise en évidence des propriétés moussantes des formulations comprenant des composés de formule (I) ou des mélanges de composés de formule (I), et des compositions (C) précédemment définies faisant l'objet de l'invention, peut être réalisée par l'évaluation du pouvoir moussant ou par l'évaluation des caractéristiques sensorielles que procure la mousse formée aux utilisateurs lors de l'opération de lavage ou de nettoyage.

**B.1** - Mise en évidence du pouvoir moussant

**B.1.1** - Principe de la méthode Ross-Miles pour l'évaluation du pouvoir moussant

Le pouvoir moussant est réalisé selon le protocole dit « de Ross Miles » issu de la norme ISO 696 et AFNOR NFT 73-404 et décrit dans le paragraphe B1.2., en diluant les bases nettoyantes et les compositions précédemment préparées au 1/10ème, de manière à représenter les conditions réelles de création de la mousse lors de la réalisation d'un shampooing par exemple.
La dilution au 1/10ème des compositions et des formulations à tester est réalisée en présence de 0.25% d'une salissure grasse (un sebum reconstitué) avec une eau déminéralisée enrichie de 3 millimoles d'ions calcium, ce qui correspond à la préparation d'une eau à 30° de dureté calcique selon la norme NFT 73-047.
La méthode consiste à mesurer le volume de mousse obtenu après la chute d'une hauteur de 450mm, de 500cm$^3$ d'une solution de composition ou de formulation à tester, sur une surface liquide d'une même solution.

**B.1.2 -** Protocole expérimental

**a)** Préparation d'une solution mère(S)

On réalise une solution mère pour chaque essai. Pour les compositions ou les formulations de tensioactifs à tester, préparer 100g d'une solution-mère (S) titrant à 10% en matière active ou en extrait sec, diluée en eau déminéralisée enrichie de 3 millimoles d'ions calcium, ce qui correspond à la préparation d'une eau à 30° de dureté calcique selon la norme NFT 73-047.

**b)** Préparation de la salissure

Dans le cas de détermination de la hauteur de mousse en présence de salissure artificielle, on pèse préalablement la quantité de salissure (0.25% soit 1.75g pour 700g de solution finale) et la quantité de solution S (soit 70g pour 700g de solution finale) nécessaires à la réalisation des mesures. Le mélange est fondu au bain-marie préchauffé à 70°C pendant 3 minutes en mélangeant à la spatule.

**c)** Préparation de la solution d'essai

On dilue le mélange [S + salissure] préalablement fondu, au 1/10ème avec de l'eau eau déminéralisée enrichie de 3 milllimoles d'ions calcium. La durée de vie des solutions d'essais est de 1 heure au maximum à partir du moment où la dilution finale a été réalisée. La solution finale d'essai est ensuite préchauffée au bain-Marie à 48°C pendant 1h minimum, puis thermostatée pendant 30 minutes pour atteindre une température de 40°C +/-1°C.

**d)** Mesure

On introduit dans une éprouvette de 50 cm$^3$ de la solution d'essai en faisant glisser le liquide le long des parois pour éviter toute formation de mousse. On place au dessus de l'éprouvette, à une hauteur fixe, une ampoule à décantée munie en son extrémité d'un embout d'acier de telle sorte que la hauteur entre la surface de la solution contenue dans l'éprouvette et l'extrémité de l'embout d'acier soit de 450 mm. On introduit environ 100 cm$^3$ de la solution d'essai dans l'ampoule à décanter, en faisant glisser le long des parois et on ajuste le niveau par écoulement jusqu'au trait de jauge en supprimant ainsi l'air présent dans le chas du robinet.
On introduit ensuite 500 cm$^3$ de solution d'essai dans l'ampoule en faisant glisser le long des parois. L'ampoule est placée sur son support et on ouvre le robinet de manière à laisser couler la solution en une seule fois dans l'éprouvette graduée avec un débit maximum. On ferme le robinet lorsque la solution atteint le trait de jauge et on déclenche alors simultanément le chronomètre. On mesure en cm la hauteur de mousse immédiatement formée au déclenchement du chronomètre puis la hauteur de mousse à 30 secondes, à 3 minutes et 5 minutes. La hauteur de mousse se mesure entre l'interface horizontale mousse/liquide et la base de l'interface mousse/air.

**B.1.3 -** Expression des résultats

Le résultat du volume de mousse à 30 secondes est exprimé en cm$^3$ en multipliant la hauteur mesurée à 30 secondes en cm par la section de l'éprouvette (30.2 cm$^2$ ou 28.3 cm$^2$ selon le modèle). La stabilité de la mousse après 5 minutes, exprimée en pourcentage, est calculée selon la formule :

[(Volume de mousse à 30s - Volume de mousse à 5min) / Volume de mousse à 30s] x 100.

**B.1.4 -** Influence des composés de formule (I) ou des mélanges de composés de formule (I) sur le pouvoir moussant de formulations comprenant des composés de formule (II).

**B.1.4.1.-** Résultats obtenus

Tableau 2 : évaluation du pouvoir moussant d'une solution contenant un composé de formule (II) et un mélange de composés de formule (I) (composition C1) et d'une solution contenant un composé de formule (II) (T1).

| Pouvoir moussant | (C1) | T1 |
|---|---|---|
| Volume de mousse à 30 secondes (cm$^3$) | 350 | 317 |
| Stabilité de la mousse à 5 minutes (%) | 94% | 83% |

**B.1.4.2.-** Analyse des résultats

Les résultats sont jugés satisfaisants lorsque la solution testée montre un volume de mousse à 30 secondes supérieur ou égal à 300 cm$^3$ et une stabilité de mousse à 5 minutes supérieure ou égale à 90% . L'exemple comparatif 1 montre qu'une solution comprenant un composé de formule (II), testée dans les conditions décrites ci-dessus, se caractérise par la génération d'une mousse abondante à 30 secondes (317cm$^3$) et par une stabilité de 83% après 5 minutes. La mise en oeuvre du procédé selon l'invention pour préparer la composition (C1), permet d'atteindre à la fois un volume de mousse après 30 secondes supérieur et également une stabilité de mousse à 5 minutes sensiblement supérieure (94% pour la composition (C1) et 83 % pour (T1)). La mise en oeuvre du procédé selon l'invention permet donc d'améliorer le pouvoir moussant de composés de formule (II).

**B.2 -** Mise en évidence des propriétés sensorielles

Parmi les caractéristiques permettant d'apprécier la qualité d'une mousse formée par une solution de tensioactif moussant, par une composition moussante et par une formulation moussante, on retiendra également des caractéristiques sensorielles que procure la mousse à l'utilisateur, et qui concernent l'appréciation de la douceur de la mousse formée, de l'aptitude à permettre un glissement facile des mains pendant le lavage (le « glissant »), de la facilité de rinçage après l'opération de lavage, de la stabilité de la mousse à la dilution par l'eau de lavage lors de cette dernière opération. Ces caractéristiques sont déterminées expérimentalement par un panel représentatif d'utilisateurs dûment entrainés, selon le protocole détaillé au paragraphe B.2.2.

**B.2.1. -** Principe de la méthode

Les propriétés sensorielles de la mousse générée par les compositions moussantes sont évaluées lors d'une opération de lavage des mains par un panel de 10 personnes, dûment entrainées à cette évaluation, qui classent sur une échelle allant de 0 à 10 : l'indice de douceur de la mousse, qui permet d'évaluer la sensation de douceur ressentie par l'utilisateur ; l'indice du glissant de la mousse, qui permet d'évaluer la facilité avec laquelle les mains glissent l'une sur l'autre pendant la phase de frottement des mains lors de l'opération de nettoyage ; l'indice de stabilité de la mousse à la dilution, qui permet d'évaluer la stabilité de la mousse contenue dans la main après les phases de frottement des mains puis l'ajout de 2cm$^3$ d'eau ; l'indice de facilité de rinçage de la mousse, qui permet d'évaluer la facilité de rincer les mains à l'eau après l'opération de frottement des mains avec le produit nettoyant sous forme de mousse.

**B.2.2.-** Protocole expérimental

Pour réaliser des mesures reproductibles, les solutions ont été conditionnées dans des flacons munis d'une pompe à mousse doseuse, qui émet directement le produit sous forme de mousse sur les mains avec une quantité fixe de produit testé. Le produit contenu dans le flacon est appliqué directement sur une des mains préalablement mouillées, par deux pressions successives de la pompe à mousse du flacon. Sur la main mouillée contenant le produit issu du flacon, l'expérimentateur frotte l'autre main à plat pendant 5 secondes, puis réalise des frottements avec les mains l'une dans l'autre pendant une durée supplémentaire de 5 secondes. La mousse est rassemblée dans une seule main pour être évaluée par l'expérimentateur qui note l'indice de douceur, l'indice du glissant de mousse. On ajoute ensuite 2cm$^3$ d'eau par l'intermédiaire d'une pipette graduée sur la main contenant la mousse rassemblée et l'expérimentateur note alors l'indice de la tenue de la mousse à la dilution. L'expérimentateur se rince ensuite les mains sous un robinet d'eau froide et évalue alors la facilité de rinçage de la mousse.

**B.2.3.-** Expression des résultats

Un indice de douceur de 0 est attribué pour une mousse très rêche et un indice de douceur de 10 est attribué pour une mousse très douce. Un indice du glissant de mousse de 0 est attribué pour une mousse qui accroche sur les mains et un indice du glissant de mousse de 10 est attribué pour une mousse très glissante sur les mains. Un indice stabilité de mousse à la dilution de 0 est attribué pour une mousse qui s'effondre lors de l'ajout des 2cm$^3$ d'eau et un indice de stabilité de mousses à la dilution de 10 est attribué pour une mousse qui se maintient parfaitement lors de l'ajout des 2cm$^3$ d'eau. Un indice de facilité de rinçage de 0 est attribué pour un rinçage des mains jugé très difficile et un indice de facilité de rinçage de 10 est attribué pour un rinçage des mains jugé très facile. Pour chaque indice, on relève les évaluations effectuées par chacun des 10 expérimentateurs et on calcule ensuite la moyenne arithmétique de chaque indice.

**B.2.4.-** Propriétés sensorielles des formulations nettoyantes et/ou détergentes obtenues par la mise en oeuvre du procédé selon l'invention et des compositions selon l'invention

**B.2.4.1.-** Résultats obtenus

Tableau 3 : évaluation des propriétés sensorielles

| Indices de propriétés sensorielles | C1 | T1 | C2 | T2 | T3 | C3 | C4 | T4 |
|---|---|---|---|---|---|---|---|---|
| Douceur | 8 | 7 | 7,7 | 5,2 | 4,0 | 5,5 | 6,0 | 3,0 |
| Glissant | 6 | 5,5 | 7,4 | 4,0 | 3,4 | 5,5 | 6,5 | 3,0 |
| Stabilité de mousse après dilution | 5 | 3 | 6,8 | 5,5 | 4,5 | 5,4 | 6,0 | 4,0 |
| Facilité de rinçage | 5,1 | 5,3 | 6,7 | 6,0 | 7,3 | 6,9 | 7,0 | 7,1 |

**B.2.4.2.-** Analyse des résultats

Les résultats sont jugés satisfaisants lorsque l'un ou plusieurs des indices de propriétés sensorielles de la formulation ou de la composition testée présente une amélioration significative, sans diminuer significativment un ou plusieurs des autres indices ; lesdits indices pris individuellement devant montrer une valeur supérieure ou égale à 5,0. Ils font apparaître une amélioration de l'une ou l'autre des propriétés sensorielles induites par les compositions selon l'invention par rapport à celles de l'état de la technique

**C) - Formulations**

Dans les formules suivantes, les pourcentages sont exprimés en poids de la formulation.

**C.1 Gel moussant visage**

Formule

| | | | |
|---|---|---|---|
| A | Lauryléther(2,2 OE) sulfate de sodium à 28% ) | 5,9 % | |
| | Eau | Qsp 100 % | |
| B | Composition (C5) selon l'invention | 5,0 % | |
| | MONTALINE™ C40 | 5,0 % | |
| | SEPITONIC™ M3 | 1,0 % | |
| | Parfum | 0,1 % | |
| | KATHON™ CG | 0,08 % | |
| C | Acide lactique | 0,15 % | |
| | Chlorure de sodium | 0,8 % | |
| D | Colorant | 0,05 % | |
| E | Chlorure de sodium | Qs | |

Mode opératoire

Préparer la phase A sous une hotte aspirante. Mélanger les ingrédients de la phase B en homogénéisant après chaque ajout. Verser ensuite A dans B. Ajouter C puis D. Ajuster la viscosité si nécessaire en ajoutant E (1.5% max.).

## C.2 Bain moussant pour enfants

Formule

| | | |
|---|---|---|
| A | ORONAL™ LCG | 10,00 % |
| | Composition (C2) selon l'invention | 13,00 % |
| | Parfum | 00,10 % |
| | SEPICIDE™ HB | 00,50 % |
| B | Eau | 20,00% |
| | CAPIGEL™ 98 | 04,50 % |
| C | Eau | QSP 100% |
| | SEPICIDE™ CI | 00,30 % |
| | Colorant | QS |
| | Hydroxyde de sodium | QS |

Mode opératoire

Mélanger ORONAL™ LCG avec le tensioactif amphotère, le parfum, et le conservateur. Diluer le CAPIGEL™ dans une partie d'eau et l'ajouter aux tensioactifs puis ajouter le reste d'eau. Ajouter le SEPICIDE™ CI, le colorant puis ajuster le pH à 7,2 environ.

## C.3 Savon liquide pour les mains

Formule

| | | |
|---|---|---|
| A | Composition (C1) | 10,00 % |
| | AMONYL™ 675SB | 10,00 % |
| | Parfum/Fragrance | 00,30 % |
| | SEPICIDE™ HB | 00,50 % |
| B | Eau | QSP 100% |
| | SEPICIDE™ CI | 00,30 % |
| | Chlorure de sodium | QS |

Mode opératoire

Mélanger les ingrédients de la phase A puis ajouter la phase B.

## C.4 Lotion purifiante concentrée

Formule

| | | |
|---|---|---|
| A | Eau | 20,00 % |
| | LIPACIDE™ UGB | 1,00 % |
| | Trométhamine | 0,75 % |

(suite)

| | | | |
|---|---|---|---|
| B | PEG120 Methyl glucose di-oléate | 5,00 % | |
| | Eau | QSP 100% | |
| | Composition (C6) selon l'invention | 29,00 % | |
| | Glycérine | 5,00 % | |
| C | Acide lactique | QS pH=4 | |

Mode opératoire

Dissoudre LIPACIDE™ UGB dans une partie de l'eau chauffée à 80°C puis ajouter la tromethamine, cette phase doit être parfaitement limpide. Fondre PEG120 methylglucose di-oléate dans le reste de l'eau préalablement chauffée à 80°C. Ajouter dans cette phase, la glycérine et la composition de l'invention : cette phase est aussi parfaitement limpide. Mélanger les 2 phases, laisser refroidir et réajuster le pH à la valeur souhaitée.

## C.5 Mousse nettoyante visage

Formule

| | | |
|---|---|---|
| A | PROTEOL™ OAT | 30 % |
| | Composition (C3) selon l'invention | 10,00 % |
| | SEPICIDE HB™ | 00,50 % |
| | Parfum | 00,20 % |
| B | Eau | QSP 100% |
| | SEPICIDE™ CI | 00.30 % |
| | SEPITONIC M3 | 01,00% |
| | Tromethamine | QS |
| | Colorant | QS |

Mode opératoire

Solubiliser le parfum et le conservateur dans le mélange des tensioactifs (A). Ajouter l'eau puis les autres ingrédients successivement.

## C.6 Shampooing anti-stress

Formule

| | | |
|---|---|---|
| A | Composition (C1) | 20,00 % |
| | SEPICIDE™ HB | 0,50 % |
| | SEPICIDE™ CI | 0,30 % |
| | Parfum | 0,30 % |
| | Eau | QSP 100% |
| | SEPICAP™ MP | 1,00 % |
| B | Eau | 10,00 % |
| | CAPIGEL™ 98 | 3,00 % |
| | Soude | QS PH=7,2 |
| | Colorant | QS |

Mode opératoire

Mélanger les ingrédients de A puis ajouter le CAPIGEL prédilué. Neutraliser.

## C.7 Lingettes nettoyantes

Formule

| A | Composition (C6) | 02,00 % |
|---|---|---|
| | AQUAXYL™ | 01,00 % |
| B | SEPICIDE™ HB$_2$ | 00,50 % |
| | Parfum | 00,05 % |
| | Hexylene glycol | 10,00 % |
| C | Eau | Qsp 100 % |

Mode opératoire

Mélanger les ingrédients de la phase B jusqu'à limpidité puis ajouter cette phase à la phase A. Ajouter C.

## C.8 Huile de bain relaxante

Formule

| Cedar wood extract | 10,00 % |
|---|---|
| Composition (C5) | 66,00 % |
| Glycérine | 24,00 % |

Mode opératoire

Mélanger l'huile essentielle la composition de l'invention jusqu'à limpidité. Ajouter ensuite la glycérine.

## C.9 Nettoyant doux à l'huile de jojoba

| A | MONTANOV™ S | 3,0% |
|---|---|---|
| | PEG-120 Methylglucose di-oléate | 2,0% |
| B | Huile de jojoba | 0,5% |
| | Diméthicone and laureth-23 and laureth-4 and salicylic acid | 2,0% |
| C | Eau | 30,0% |
| | Glycérine | 3,0% |
| | Polyquaternium™ 10 | 0,7% |
| D | SOMEPON™ T25 | 4,5% |
| | Composition (C6) | 30,0% |
| | Eau | 5,0% |
| | SEPICIDE™ CI | 0,2% |
| | SEPICIDE™ HB | 0,3% |
| | Parfum | 0,1% |
| E | Eau | qs 100% |
| | CAPIGEL™ 98 | 2,0% |

(suite)

| Soude | qs PH = 7 |
|---|---|

Mode opératoire

Disperser le Polyquaternium™10 de la phase C dans le mélange eau + glycérine. Chauffer à 70°C et ajouter les ingrédients de A puis de B. Emulsionner. Démarrer le refroidissement. A 60°C ajouter les tensioactifs un par un (D). A 30°C ajouter le CAPIGEL™ 98 pré-dilué (E). Puis au final neutraliser avec la soude.

## C.10 Savon liquide antibactérien

| | |
|---|---|
| Composition (C6) | 15,00 % |
| Digluconate de chlorhexidine | 00,20 % |
| ORAMIDE™ DL 200 AF | 03,00 % |
| Eau | QSP100 |
| Parfum | 00,05 % |
| Colorant | QS |
| Chlorure de sodium | QS |

Mode opératoire

Ajouter et mélanger les constituants dans l'ordre indiqué.

## C11 Shampooing conditionneur.

| | | |
|---|---|---|
| A | Cetyl trimethyl ammonium chloride | 03,50 % |
| | Composition (C5) | 25,00 % |
| | Dimethicone copolyol | 01,00 % |
| | Parfum | 00,50 % |
| | AMONYL™ 380BA | 11,00 % |
| | KATHON™ CG | 0,08 % |
| B | Polyquaternium™10 | 00,30 % |
| | Acide lactique | QS . |
| | Eau | QSP 100 % |
| | Colorant | QS |
| | Acide lactique | QS PH=6 |

Mode opératoire

Préparer séparément la phase B: mélanger jusqu'à limpidité. Mélanger soigneusement les ingrédients dans l'ordre indiqué.

## C12 Poudre de bain

| | | |
|---|---|---|
| A | MICROPEARL™ M | 5.00 % |
| | Mica | 72.00 % |
| | Pigment | 3.00 % |
| B | Rosmarinus officinalis (Rosemary) leaf extract | 10.00 % |

(suite)

| Composition (C5) | 10.00 % |
|---|---|

Mode opératoire

Broyer les poudres de la phase A. Solubiliser l'huile essentielle avec la composition 4 selon l'invention (B) et introduire lentement cette phase B dans le broyeur en laissant tourner 6 minutes après la fin de l'introduction de la phase liquide.

Les caractéristiques des produits utilisés dans les exemples précédents, sont les suivantes :

Le SEPITONIC™ M3, mélange d'aspartate de magnésium, de gluconate de zinc et de gluconate de cuivre, est un actif énergisant commercialisé par la société SEPPIC.

Le Polyquaternium™ 10 est un sel d'ammonium quaternaire d'une hydroxyéthylcellulose, commercialisé par la société AMERCHOL sour l'appelation UCARE POLYMER™ JR - 400.

Le KATHON™ CG, mélange de méthylchloroisothiazolinone et de méthylisothiazolinone, est un agent conservateur commercialisé par la société ROHM & HAAS.

L'ORONAL™ LCG, mélange de PEG-40 glyceryl cocoate et de coceth-sulfate de sodium, est un agent moussant commercialisé par la société SEPPIC.

L'AMONYL™ 675SB est une Cocoamidopropyl hydroxy sultaïne, commercialisée par la société SEPPIC.

Le SEPICIDE™ HB, un mélange de phénoxyéthanol, de méthylparaben, d'éthylparaben, de propylparaben et de butylparaben, est un agent conservateur commercialisé par la société SEPPIC.

Le CAPIGEL™ 98 est un copolymère d'acrylates commercialisé par la société SEPPIC.

Le SEPICIDE™ CI, imidazoline urée, est un agent conservateur commercialisé par la société SEPPIC.

Le SEPICIDE™ HB2, un mélange de phénoxyéthanol, de méthylparaben, d'éthylparaben, de propylparaben, de butylparaben et d'isobutylparaben, est un agent conservateur commercialisé par la société SEPPIC.

Le PROTEOL™ OAT est un mélange de N-lauryl aminoacides obtenus par hydrolyse totale de protéine d'avoine tel que décrit dans WO 94/26694 et commercialisé par la société SEPPIC.

La MONTALINE™ C40 est un sel de chlorure de Cocamidopropyl betaïnamide de Monoéthanolamine.

Le MONTANOV™ S (alcools C12-C18 et cocoglucosides), est une composition auto-émulsionnable telle que celles décrites dans EP 0 729 382, commercialisée par la société SEPPIC.

Le SEPICAP™ MP, est un mélange de N-cocoyl aminoacides et de sel de potassium de diméthicone PEG-7 de panthényl phosphate, commercialisé par la société SEPPIC.

L'AQUAXYL™ est un actif hydratant, comprenant un mélange de Xylitylpolyglucosides, d'anhydroxylitol et de xylitol, commercialisé par la société SEPPIC.

Le SOMEPON™ T25 est un methyl cocoyl taurate de sodium, commercialisé par la société SEPPIC.

Le LIPACIDE™ UGB est de l'Undecylenoyl glycine commercialisé par la société SEPPIC.

L'ORAMIDE™ DL 200 AF est une Cocamide de Di Ethanol Amide, commercialisée par la société SEPPIC.

L'AMONYL™ 380BA est une Cocamidopropyl betaïne, commercialisée par la société SEPPIC.

Le MICROPEARL™ M est un polymère polyméthylméthacrylate réticulé se présentant sous forme de poudre et utilisé comme modificateur de texture.

## Revendications

1. Procédé pour améliorer les propriétés moussantes d'une formulation nettoyante et/ou détergente à usage topique, **caractérisé en ce que** l'on incorpore dans ladite formulation, une quantité efficace d'un composé de formule (I):

$$R_1\text{-}O\text{-}(G)_x\text{-}H \qquad (1)$$

dans laquelle :

x représente un nombre décimal compris entre 1 et 5,
G représente le reste d'un sucre réducteur et
$R_1$ représente un radical monovalent de formule (A) :

$$\text{-}CH_2\text{-}(CHOH)_n\text{-}CH_2\text{-}OH \qquad (A)$$

dans laquelle n est un nombre entier égal à 2, 3 ou 4, ou bien
R$_1$ représente un radical monovalent de formule (B) :

$$-(CH_2-CHOH-CH_2-O)_m-H \qquad (B)$$

dans laquelle m est un nombre entier égal à 1, 2 ou 3,
ou d'un mélange de composés de formules (I).

2. Procédé tel que défini à la revendication 1, **caractérisé en ce que** dans la formule (I), G représente le reste d'un sucre réducteur choisi parmi le glucose, le dextrose, le saccharose, le fructose, l'idose, le gulose, le galactose, le maltose, l'isomaltose, le maltotriose, le lactose, le cellobiose, le mannose, le ribose, le xylose, l'arabinose, le lyxose, l'allose, l'altrose, le dextrane ou le tallose.

3. Procédé tel que défini à la revendication 2 **caractérisé en ce que** dans la formule (I), G représente le reste d'un sucre réducteur choisi parmi le glucose, le xylose et l'arabinose.

4. Procédé tel que défini à l'une des revendications 1 à 3, **caractérisé en ce que** ladite formulation nettoyante et/ou détergente à usage topique comprend au moins un agent tensioactif moussant et/ou détergent.

5. Procédé tel que défini à la revendication 4, **caractérisé en ce que** l'agent tensioactif moussant et/ou détergent est un composé de formule (II) :

$$R_2-[O-(CH_2-CH_2-O)_pSO_3]_rX \qquad (II)$$

dans laquelle:

R$_2$ représente un radical hydrocarboné aliphatique, saturé ou insaturé, linéaire ou ramifié, comportant de 6 à 22 atomes de carbone,
p représente un nombre décimal compris entre 1 et 10, de préférence entre 2 et 4,
r est un nombre entier égal à 1 ou à 2 et
X représente le cation d'un métal alcalin ou d'un métal alcalino-terreux, l'ion ammonium, l'ion hydroxyéthyl ammonium, l'ion tris(hydroxyéthyl) ammonium ou un mélange de composés de formule (II).

6. Procédé tel que défini à la revendication 4, **caractérisé en ce que** l'agent tensioactif moussant et/ou détergent est un composé de formule (III) :

$$R_3-O-(S)_y-H \qquad (III)$$

dans laquelle :

y représente un nombre décimal compris entre 1 et 5,
S représente le reste d'un sucre réducteur et
R$_3$ représente un radical alkyle linéaire ou ramifié, saturé ou insaturé, ayant de 8 à 16 atomes de carbone, de préférence de 8 à 14 atomes de carbone.

7. Procédé tel que défini à l'une quelconque des revendications 4 à 6, **caractérisé en ce que** le rapport massique, composé de formule (I) sur agent tensioactif moussant et/ou détergent présents dans ladite formulation à usage topique, est compris entre 1/30 et 10/1, et plus particulièrement entre 1/30 et 1/1.

8. Composition (C) **caractérisée en ce qu'**elle comprend pour 100% de sa masse :

de 97% à 40% massique d'un composé de formule (III) :
R$_3$-O-(S)$_y$-H (III)

dans laquelle :

y représente un nombre décimal compris entre 1 et 5,
S représente le reste d'un sucre réducteur et

$R_3$ représente un radical alkyle linéaire ou ramifié, saturé ou insaturé, ayant de 8 à 16 atomes de carbone, de préférence de 8 à 14 atomes de carbone.

de 1 % à 25% massique d'un composé de formule (I) :

$$R_1\text{-O-}(G)_x\text{-H} \qquad (I)$$

dans laquelle :

x représente un nombre décimal compris entre 1 et 5,
G représente le reste d'un sucre réducteur et
$R_1$ représente un radical monovalent de formule (A) :

$$-CH_2\text{-}(CHOH)_n\text{-}CH_2\text{-OH} \qquad (A)$$

dans laquelle n est un nombre entier égal à 2, 3 ou 4, ou bien
$R_1$ représente un radical monovalent de formule (B) :

$$-(CH_2\text{-CHOH-}CH_2\text{-O})_m\text{-H} \qquad (B)$$

dans laquelle m est un nombre entier égal à 1, 2 ou 3,

ou d'un mélange de composés de formules (I) et,
jusqu'à 50% massique d'un solvant topiquement acceptable.

9. Composition (C) telle que définie à la revendication 8, **caractérisée en ce que** dans la formule (I), G représente le reste d'un sucre réducteur choisi parmi le glucose, le xylose et l'arabinose.

10. Composition (C) telle que définie à l'une quelconque des revendications 8 ou 9, **caractérisée en ce que** dans la formule (III), $R_3$ représente un radical hydrocarboné aliphatique saturé choisi parmi le radical n-octyle, le radical n-décyle, le radical n-dodécyle, le radical n-tétradécyle, le radical n-hexadécyle.

11. Composition (C) telle que définie à l'une quelconque des revendications 8 à 10, pour laquelle :

dans la formule (I) G représente le reste du glucose, du xylose ou de l'arabinose, et $R_1$ représente un radical monovalent de formule (A) pour lequel n est égal à 2 ou à 3, ou un radical monovalent de formule (B) pour lequel m est égal à 1 ou à 2.
dans la formule (III), S représente le reste du glucose ou du xylose, et $R_3$ représente un radical choisi parmi les radicaux n-octyle, n-décyle, n-dodécyle, n-tétradécyle, n-hexadécyle.

12. Composition (C) telle que définie à l'une quelconque des revendications 8 à 11, **caractérisée en ce qu'**elle comprend un solvant topiquement acceptable choisi parmi un ou plusieurs éléments d'un groupe constitué par l'eau, des glycols, des polyols, des alcools, des polyols alcoxylés, des éthers de glycols.

13. Procédé de préparation d'une composition (C) telle que définie à l'une quelconque des revendications 8 à 12 **caractérisé en ce qu'**il comprend :

une étape (a) de mélange, sous agitation, d'un composé de formule (I) ou d'un mélange de composés de formule (I) avec un composé de formule (III) ou un mélange de composés de formules (III) et si nécessaire,
une étape (b) de mélange sous agitation de la combinaison préparée à l'étape (a), avec un solvant topiquement acceptable.

14. Utilisation d'un composé de formule (I)

$$R_1\text{-O-}(G)_x\text{-H} \qquad (I)$$

dans laquelle :

x représente un nombre décimal compris entre 1 et 5,
G représente le reste d'un sucre réducteur et
R$_1$ représente un radical monovalent de formule (A) :

$$-CH_2-(CHOH)_n-CH_2-OH \qquad (A)$$

dans laquelle n est un nombre entier égal à 2, 3 ou 4, ou bien
R$_1$ représente un radical monovalent de formule (B) :

$$-(CH_2-CHOH-CH_2-O)_m-H \qquad (B)$$

dans laquelle m est un nombre entier égal à 1, 2 ou à 3,

ou d'un mélange de composés de formules (I), pour améliorer les propriétés moussantes d'une formulation à usage topique.

**15.** Utilisation d'une composition (C) telle que définie à l'une quelconque des revendications 8 à 12, pour améliorer les propriétés moussantes d'une formulation à usage topique.

**16.** Préparation d'une formulation nettoyante et/ou moussante à usage topique **caractérisée en ce qu'**on met en oeuvre une quantité efficace d'une composition (C) telle que définie à l'une quelconque des revendications 8 à 12.

**Claims**

**1.** Process for improving the foaming properties of a cleansing and/or detergent formulation for topical use, **characterized in that** an effective amount of a compound of formula (I):

$$R_1-O-(G)_x-H \qquad (I)$$

in which:

x represents a decimal number between 1 and 5,
G represents a reducing sugar residue, and
R$_1$ represents a monovalent radical of formula (A):

$$-CH_2-(CHOH)_n-CH_2-OH \qquad (A)$$

in which n is an integer equal to 2, 3 or 4, or alternatively
R$_1$ represents a monovalent radical of formula (B):

$$-(CH_2-CHOH-CH_2-O)_mH \qquad (B)$$

in which m is an integer equal to 1, 2 or 3,

or a mixture of compounds of formula (I), is incorporated into said composition.

**2.** Process as defined in Claim 1, **characterized in that**, in formula (I), G represents a reducing sugar residue chosen from glucose, dextrose, sucrose, fructose, idose, gulose, galactose, maltose, isomaltose, maltotriose, lactose, cellobiose, mannose, ribose, xylose, arabinose, lyxose, allose, altrose, dextran and talose.

**3.** Process as defined in Claim 2, **characterized in that**, in formula (I), G represents a reducing sugar residue chosen from glucose, xylose and arabinose.

**4.** Process as defined in one of Claims 1 to 3, **characterized in that** said cleansing and/or detergent formulation for topical use comprises at least one foaming and/or detergent surfactant.

**5.** Process as defined in Claim 4, **characterized in that** the foaming and/or detergent surfactant is a compound of

formula (II):

$$R_2\text{-}[O\text{-}(CH_2\text{-}CH_2\text{-}O)_p SO_3]_r X \qquad (II)$$

in which:

R$_2$ represents a saturated or unsaturated, linear or branched aliphatic hydrocarbon-based radical containing from 6 to 22 carbon atoms,
p represents a decimal number between 1 and 10 and preferably between 2 and 4,
r is an integer equal to 1 or 2, and
X represents the cation of an alkali metal or of an alkaline-earth metal, the ammonium ion, the hydroxyethylammonium ion or the tris(hydroxyethyl)-ammonium ion, or a mixture of compounds of formula (II) .

6. Process as defined in Claim 4, **characterized in that** the foaming and/or detergent surfactant is a compound of formula (III):

$$R_3\text{-}O\text{-}(S)_y\text{-}H \qquad (III)$$

in which:

y represents a decimal number between 1 and 5,
S represents a reducing sugar residue, and
R$_3$ represents a linear or branched, saturated or unsaturated alkyl radical containing from 8 to 16 carbon atoms and preferably from 8 to 14 carbon atoms.

7. Process as defined in any one of Claims 4 to 6, **characterized in that** the mass ratio of compound of formula (I) to foaming and/or detergent surfactant present in said formulation for topical use is between 1/30 and 10/1 and more particularly between 1/30 and 1/1.

8. Composition (C), **characterized in that** it comprises, per 100% of its mass:

- from 97% to 40% by mass of a compound of formula (III):

$$R_3\text{-}O\text{-}(S)_y\text{-}H \qquad (III)$$

in which:

y represents a decimal number between 1 and 5,
S represents a reducing sugar residue, and
R$_3$ represents a linear or branched, saturated or unsaturated alkyl radical containing from 8 to 16 carbon atoms and preferably from 8 to 14 carbon atoms,

- from 1% to 25% by mass of a compound of formula (I):

$$R_1\text{-}O\text{-}(G)_x\text{-}H \qquad (I)$$

in which:

x represents a decimal number between 1 and 5,
G represents a reducing sugar residue, and
R$_1$ represents a monovalent radical of formula (A):

$$\text{-}CH_2\text{-}(CHOH)_n\text{-}CH_2\text{-}OH \qquad (A)$$

in which n is an integer equal to 2, 3 or 4, or alternatively
R$_1$ represents a monovalent radical of formula (B):

$$\text{-}(CH_2\text{-}CHOH\text{-}CH_2\text{-}O)_m\text{-}H \qquad (B)$$

in which m is an integer equal to 1, 2 or 3,
or a mixture of compounds of formula (I); and up to 50% by mass of a topically acceptable solvent.

9. Composition (C) as defined in Claim 8, **characterized in that**, in formula (I), G represents a reducing sugar residue chosen from glucose, xylose and arabinose.

10. Composition (C) as defined in either of Claims 8 and 9, **characterized in that**, in formula (III), $R_3$ represents a saturated aliphatic hydrocarbon-based radical chosen from the n-octyl radical, the n-decyl radical, the n-dodecyl radical, the n-tetradecyl radical and the n-hexadecyl radical.

11. Composition (C) as defined in any one of Claims 8 to 10, for which:

in formula (I), G represents a glucose, xylose or arabinose residue, and $R_1$ represents a monovalent radical of formula (A) for which n is equal to 2 or 3, or a monovalent radical of formula (B) for which m is equal to 1 or 2, in formula (III), S represents a glucose or xylose residue and $R_3$ represents a radical chosen from n-octyl, n-decyl, n-dodecyl, n-tetradecyl and n-hexadecyl radicals.

12. Composition (C) as defined in any one of Claims 8 to 11, **characterized in that** it comprises a topically acceptable solvent chosen from one or more elements of a group constituted by water, glycols, polyols, alcohols, alkoxylated polyols and glycol ethers.

13. Process for preparing a composition (C) as defined in any one of Claims 8 to 12, **characterized in that** it comprises:

<u>a step (a)</u> of mixing, with stirring, a compound of formula (I) or a mixture of compounds of formula (I) with a compound of formula (III) or a mixture of compounds of formula (III) and, if necessary,
<u>a step (b)</u> of mixing, with stirring, the combination prepared in step (a), with a topically acceptable solvent.

14. Use of a compound of formula (I)

$$R_1\text{-O-}(G)_x\text{-H} \qquad (I)$$

in which:

x represents a decimal number between 1 and 5,
G represents a reducing sugar residue, and
$R_1$ represents a monovalent radical of formula (A):

$$-CH_2\text{-}(CHOH)_n\text{-}CH_2\text{-OH} \qquad (A)$$

in which n is an integer equal to 2, 3 or 4, or alternatively
$R_1$ represents a monovalent radical of formula (B):

$$-(CH_2\text{-CHOH-}CH_2\text{-O})_m H \qquad (B)$$

in which m is an integer equal to 1, 2 or 3,
or a mixture of compounds of formula (I), for improving the foaming properties of a formulation for topical use.

15. Use of a composition (C) as defined in any one of Claims 8 to 12, for improving the foaming properties of a formulation for topical use.

16. Preparation of a cleansing and/or foaming formulation for topical use, **characterized in that** an effective amount of composition (C) as defined in any one of Claims 8 to 12 is used.

**Patentansprüche**

1. Verfahren zur Verbesserung der Schäumungseigenschaften einer Reinigungs- und/oder Waschmittelformulierung zur topischen Anwendung, **dadurch gekennzeichnet, daß** man in die Formulierung eine wirksame Menge einer Verbindung der Formel (I):

$$R_1\text{-O-}(G)_x\text{-H} \qquad \text{(I)}$$

worin:

x für eine Dezimalzahl zwischen 1 und 5 steht,
G für den Rest eines reduzierenden Zuckers steht und
$R_1$ für einen einwertigen Rest der Formel (A):

$$-CH_2\text{-}(CHOH)_n\text{-}CH_2\text{-OH} \qquad \text{(A)}$$

worin n eine ganze Zahl mit einem Wert von 2, 3 oder 4 ist, steht oder
$R_1$ für einen einwertigen Rest der Formel (B):

$$-(CH_2\text{-CHOH-}CH_2\text{-O})_m H \qquad \text{(B)}$$

worin m eine ganze Zahl mit einem Wert von 1, 2 oder 3 ist, steht,

oder eine Mischung von Verbindungen der Formel (I) einarbeitet.

2. Verfahren nach Anspruch 1, **dadurch gekennzeichnet, daß** in Formel (I) G für den Rest eines reduzierenden Zuckers, der unter Glucose, Dextrose, Saccharose, Fructose, Idose, Gulose, Galactose, Maltose, Isomaltose, Maltotriose, Lactose, Cellobiose, Mannose, Ribose, Xylose, Arabinose, Lyxose, Allose, Altrose, Dextran und Talose ausgewählt ist, steht.

3. Verfahren nach Anspruch 1, **dadurch gekennzeichnet, daß** in Formel (I) G für den Rest eines reduzierenden Zuckers, der unter Glucose, Xylose und Arabinose ausgewählt ist, steht.

4. Verfahren nach einem der Ansprüche 1 bis 3, **dadurch gekennzeichnet, daß** die Reinigungs-und/oder Waschmittelformulierung zur topischen Anwendung mindestens ein schäumendes und/oder waschaktives Tensid enthält.

5. Verfahren nach Anspruch 4, **dadurch gekennzeichnet, daß** es sich bei dem schäumenden und/oder waschaktiven Tensid um eine Verbindung der Formel (II):

$$R_2\text{-}[O\text{-}(CH_2\text{-}CH_2\text{-O})_p SO_3]_r X \qquad \text{(II)}$$

worin:

$R_2$ für einen linearen oder verzweigten, gesättigten oder ungesättigten aliphatischen Kohlenwasserstoffrest mit 6 bis 22 Kohlenstoffatomen steht,
p für eine Dezimalzahl zwischen 1 und 10 und vorzugsweise zwischen 2 und 4 steht,
r eine ganze Zahl mit einem Wert von 1 oder 2 ist und
X für das Kation eines Alkalimetalls oder Erdalkalimetalls, das Ammoniumion, das Hydroxyethylammoniumion oder das Tris(hydroxyethyl)-ammoniumion steht,

oder eine Mischung von Verbindungen der Formel (II) handelt.

6. Verfahren nach Anspruch 4, **dadurch gekennzeichnet, daß** es sich bei dem schäumenden und/oder waschaktiven Tensid um eine Verbindung der Formel (III):

$$R_3\text{-O-}(S)_y\text{-H} \qquad \text{(III)}$$

worin:

y für eine Dezimalzahl zwischen 1 und 5 steht,

S für den Rest eines reduzierenden Zuckers steht und

$R_3$ für einen gesättigten oder ungesättigten, linearen oder verzweigten Alkylrest mit 8 bis 16 Kohlenstoffatomen und vorzugsweise 8 bis 14 Kohlenstoffatomen steht,

handelt.

7. Verfahren nach einem der Ansprüche 4 bis 6, **dadurch gekennzeichnet, daß** das Massenverhältnis von Verbindung der Formel (I) zu schäumendem und/oder waschaktivem Tensid in der Formulierung zur topischen Anwendung zwischen 1/30 und 10/1 und spezieller zwischen 1/30 und 1/1 liegt.

8. Zusammensetzung (Z), **dadurch gekennzeichnet, daß** sie auf 100% ihrer Masse: 97 bis 40 Masse-% einer Verbindung der Formel (III):

$$R_3\text{-O-}(S)_y\text{-H} \qquad (III)$$

worin:

y für eine Dezimalzahl zwischen 1 und 5 steht,

S für den Rest eines reduzierenden Zuckers steht und

$R_3$ für einen gesättigten oder ungesättigten, linearen oder verzweigten Alkylrest mit 8 bis 16 Kohlenstoffatomen und vorzugsweise 8 bis 14 Kohlenstoffatomen steht,

1 bis 25 Masse-% einer Verbindung der Formel (I):

$$R_1\text{-O-}(G)_x\text{-H} \qquad (I)$$

worin:

x für eine Dezimalzahl zwischen 1 und 5 steht,

G für den Rest eines reduzierenden Zuckers steht und

$R_1$ für einen einwertigen Rest der Formel (A):

$$\text{-CH}_2\text{-(CHOH)}_n\text{-CH}_2\text{-OH} \qquad (A)$$

worin n eine ganze Zahl mit einem Wert von 2, 3 oder 4 ist, steht oder

$R_1$ für einen einwertigen Rest der Formel (B):

$$\text{-(CH}_2\text{-CHOH-CH}_2\text{-O)}_m\text{H} \qquad (B)$$

worin m eine ganze Zahl mit einem Wert von 1, 2 oder 3 ist, steht,

oder einer Mischung von Verbindungen der Formel (I) und

bis zu 50 Masse-% eines topisch unbedenklichen Lösungsmittels enthält.

9. Zusammensetzung (Z) nach Anspruch 8, **dadurch gekennzeichnet, daß** in Formel (I) G für den Rest eines reduzierenden Zuckers, der unter Glucose, Xylose und Arabinose ausgewählt ist, steht.

10. Zusammensetzung (Z) nach einem der Ansprüche 8 oder 9, **dadurch gekennzeichnet, daß** in Formel (III) $R_3$ für einen gesättigten aliphatischen Kohlenwasserstoffrest, der unter dem n-Octylrest, dem n-Decylrest, dem n-Dodecylrest, dem n-Tetradecylrest und dem n-Hexadecylrest ausgewählt ist, steht.

11. Zusammensetzung (Z) nach einem der Ansprüche 8 bis 10, für die:

in Formel (I) G für den Rest von Glucose, Xylose oder Arabinose steht und $R_1$ für einen einwertigen Rest der Formel (A), für den n gleich 2 oder 3 ist, oder einen einwertigen Rest der Formel (B), für den m gleich 1 oder 2 ist, steht,

in Formel (III) S für den Rest von Glucose oder Xylose steht und $R_3$ für einen Rest, der unter n-Octyl-, n-Decyl-, n-Dodecyl-, n-Tetradecyl- und n-Hexadecylresten ausgewählt ist, steht.

12. Zusammensetzung (Z) nach einem der Ansprüche 8 bis 11, **dadurch gekennzeichnet, daß** sie ein topisch unbedenkliches Lösungsmittel, das unter einem oder mehreren Elementen einer aus Wasser, Glykolen, Polyolen, Alkoholen, alkoxylierten Polyolen und Glykolethern bestehenden Gruppe ausgewählt ist, enthält.

13. Verfahren zur Herstellung einer Zusammensetzung (Z) nach einem der Ansprüche 8 bis 12, **dadurch gekennzeichnet, daß** man:

in einem Schritt (a) eine Verbindung der Formel (I) oder eine Mischung von Verbindungen der Formel (I) unter Rühren mit einer Verbindung der Formel (III) oder einer Mischung von Verbindungen der Formel (III) mischt und erforderlichenfalls

in einem zweiten Schritt (b) die in Schritt (a) hergestellte Kombination unter Rühren mit einem topisch unbedenklichen Lösungsmittel mischt.

14. Verwendung einer Verbindung der Formel (I)

$$R_1\text{-}O\text{-}(G)_x\text{-}H \qquad (I)$$

worin:

x für eine Dezimalzahl zwischen 1 und 5 steht,
G für den Rest eines reduzierenden Zuckers steht und
$R_1$ für einen einwertigen Rest der Formel (A):

$$-CH_2\text{-}(CHOH)_n\text{-}CH_2\text{-}OH \qquad (A)$$

worin n eine ganze Zahl mit einem Wert von 2, 3 oder 4 ist, steht oder
$R_1$ für einen einwertigen Rest der Formel (B):

$$-(CH_2\text{-}CHOH\text{-}CH_2\text{-}O)_m H \qquad (B)$$

worin m eine ganze Zahl mit einem Wert von 1, 2 oder 3 ist, steht,

oder einer Mischung von Verbindungen der Formel (I) zur Verbesserung der Schäumungseigenschaften einer Formulierung zur topischen Anwendung.

15. Verwendung einer Zusammensetzung (Z) nach einem der Ansprüche 8 bis 12 zur Verbesserung der Schäumungseigenschaften einer Formulierung zur topischen Anwendung.

16. Herstellung einer Reinigungsmittelformulierung und/oder schäumenden Formulierung zur topischen Anwendung, **dadurch gekennzeichnet, daß** man eine wirksame Menge einer Zusammensetzung (Z) nach einem der Ansprüche 8 bis 12 verwendet.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2668080
- FR 2734496
- FR 2756195
- FR 2762317
- FR 2784680
- FR 2784904
- FR 2791565
- FR 2790977
- FR 2807435
- FR 2804432
- FR 2830774
- FR 2830445
- FR 2852257
- FR 2858554
- FR 2820316
- FR 2852258
- WO 9426694 A
- EP 0729382 A

**Littérature non-brevet citée dans la description**

- **Daniel Voet ; Judith G. Voet.** Biochemistry. John Wyley & Sons, 1990, 250